# EUROPEAN PATENT APPLICATION

(11) **EP 4 053 188 A1**
(43) Date of publication of application: **07.09.2022**
(21) Application number: 20883352.5
(22) Date of filing: 27.10.2020
(51) Int. Cl.: C08G 69/44

(54) **LIQUID-CRYSTAL POLYESTER POWDER AND METHOD FOR PRODUCING LIQUID-CRYSTAL POLYESTER SOLUTION COMPOSITION**

(30) Priority: 31.10.2019 JP 2019198458
(71) Applicant: SUMITOMO CHEMICAL COMPANY LIMITED, Tokyo 103-6020 (JP)
(72) Inventor: AZAMI Shohei, Tsukuba-shi, Ibaraki 300-3294 (JP); ITO Toyonari, Tokyo 103-6020 (JP)
(74) Representative: Vossius & Partner Patentanwälte Rechtsanwälte mbB
(86) International application number: PCT/JP2020/040224
(87) International publication number: WO 2021/085412

(57) **Abstract**

The present invention provides: a liquid-crystal polyester solution composition which changes little in solution viscosity with the flow initiation temperature of the liquid-crystal polyester powder, and a liquid-crystal polyester powder useful for the liquid-crystal polyester solution composition. This liquid-crystal polyester powder is soluble in aprotic solvents and is characterized in that the proportion of particles having a particle diameter, as determined by dry sieving test according to JIS K 0069 (1992), of less than 250 µm is 23.5 mass% or less. The present invention further provides a method for producing the liquid-crystal polyester solution composition, the method comprising dissolving the liquid-crystal polyester powder in an aprotic solvent to obtain the liquid-crystal polyester solution composition.

## Description

### TECHNICAL FIELD

The present invention relates to a liquid crystal polyester powder, and a method for producing a liquid crystal polyester solution composition.

The present invention claims priority on the basis of Japanese Patent Application No. 2019-198458, filed in Japan on October 31, 2019, the contents of which are incorporated herein by reference.

### BACKGROUND OF THE INVENTION

A liquid crystal polyester is produced by subjecting feedstock monomers corresponding to repeating units (structural units) constituting the liquid crystal polyester to melt-polymerization, followed by subjecting the resultant polymer (prepolymer) to solid-phase polymerization. The thus produced liquid crystal polyester is widely used as an electronic substrate material because it exhibits excellent high-frequency characteristics and low hygroscopicity.

In recent years, attention has been paid to the application of a liquid crystal polyester film produced by extrusion-molding using such a liquid crystal polyester as a feedstock material to an insulating film in a multilayer printed circuit board or a flexible printed wiring board.

However, there is a problem in that the liquid crystal polyester film is remarkably oriented in the extrusion direction, and therefore the anisotropy in the horizontal direction (direction perpendicular to the extrusion direction) is larger than that in the vertical direction (extrusion direction) and the mechanical strength tends to be low.

In response to such a problem, the use of a solution composition including: a liquid crystal polyester powder having a structural unit derived from an aromatic amine derivative; and an aprotic solvent has been conventionally proposed as a technique for providing a liquid crystal polyester film having low anisotropy (see Patent Document 1).

### DOCUMENTS OF RELATED ART

### Patent Documents

Patent Document 1: Japanese Patent Publication No. 4470390

### SUMMARY OF THE INVENTION

### PROBLEMS TO BE SOLVED BY THE INVENTION

However, there is a case in which the solution viscosity of a liquid crystal polyester solution composition including a liquid crystal polyester powder and an aprotic solvent changes significantly when the liquid crystal polyester powder has a high flow starting temperature. In such a case, there is a problem in that the controllability of the solution viscosity is poor.

The present invention has been made in view of such circumstances, and aims to provide a liquid crystal polyester solution composition in which a change in solution viscosity for flow starting temperature of the liquid crystal polyester powder is suppressed, and the liquid crystal polyester powder useful therein.

### MEANS TO SOLVE THE PROBLEMS

In order to solve the above-mentioned problems, the present invention adopts the following constitution.

One aspect of the present invention is a liquid crystal polyester powder soluble in an aprotic solvent, characterized in that the proportion of particles having a particle size of less than 250 µm is 23.5% by mass or less, the particle size being measured by a dry sieving test method in accordance with JIS K 0069 (1992).

In the liquid crystal polyester powder of one aspect, the center particle size (D50) measured by the dry sieving test method is preferably 650 µm to 1200 µm.

In the liquid crystal polyester powder of one aspect, the proportion of particles having a particle size of less than 250 µm is preferably 11.5% by mass or more, the particle size being measured by the dry sieving test method.

In the liquid crystal polyester powder of one aspect, the proportion of particles having a particle size of less than 106 µm is preferably 10.0% by mass or less, the particle size being measured by the dry sieving test.

In the liquid crystal polyester powder of one aspect, the proportion of particles having a particle size of less than 106 µm is preferably 1.5% by mass or more, the particle size being measured by the dry sieving test.

It is preferable that the liquid crystal polyester powder of one aspect include a liquid crystal polyester having a structural unit (u1) of the following formula (1), a structural unit (u2) of the following formula (2) and a structural unit (u3) of the following formula (3), the amount of the structural unit (u1) be 30% by mol to 80% by mol, the amount of the structural unit (u2) be 10% by mol to 35% by mol, and the amount of the structural unit (u3) be 10% by mol to 35% by mol relative to the total structural units constituting the liquid crystal polyester.

-O-Ar¹-CO- (1)

-CO-Ar²-CO- (2)

-X- Ar³-Y- (3)

In the formulae (1) to (3), Ar¹ is a 1,4-phenylene group, a 2,6-naphthalenediyl group or a 4,4'-biphenylylene group. Ar² is a 1,4-phenylene group, a 1,3-phenylene group or a 2,6-naphthalenediyl group. Ar³ is a 1,4-phenylene group or a 1,3-phenylene group. X is -NH-. Y is -O- or -NH-. Any of the hydrogen atoms in Ar¹, Ar² or Ar³ may be each independently substituted with a halogen atom, an alkyl group having a carbon number of 1 to 10 or an aryl group having a carbon number of 6 to 20.

Another aspect of the present invention is a method for producing a liquid crystal polyester solution composition, characterized in that the above-mentioned liquid crystal polyester powder is dissolved in an aprotic solvent to obtain the liquid crystal polyester solution composition.

### EFFECTS OF THE INVENTION

One aspect of the present invention makes it possible to provide a liquid crystal polyester powder having a capability of suppressing a change in solution viscosity of a liquid crystal polyester solution composition for the flow starting temperature of the liquid crystal polyester powder.

In addition, another aspect of the present invention makes it possible to provide a liquid crystal polyester solution composition in which a change in solution viscosity for flow starting temperature of the liquid crystal polyester powder is suppressed.

### EMBODIMENTS FOR CARRYING OUT THE INVENTION

### (Liquid crystal polyester powder)

A liquid crystal polyester powder according to the present embodiment is a liquid crystal polyester powder soluble in an aprotic solvent, in which the proportion of particles having a particle size of less than 250 µm is 23.5% by mass or less, the particle size being measured by a dry sieving test method in accordance with JIS K 0069 (1992).

The following test method makes it possible to confirm that a liquid crystal polyester powder is "soluble in an aprotic solvent".

### Test method

A liquid crystal polyester powder in an aprotic solvent is stirred for 1 hour to 6 hours at a temperature between 120°C and 180°C, followed by cooling the resultant to room temperature (23°C). Then, the resultant is subjected to filtration using a pressurized filtering-machine with a 5 µm membrane filter, followed by observing the residue on the membrane filter. When no solid product is observed at the time, it is determined that the liquid crystal polyester powder is soluble in an aprotic solvent.

Further specifically, 1 part by mass of a liquid crystal polyester powder is stirred in 99 parts by mass of an aprotic solvent at 140°C for 4 hours, followed by cooling to 23°C. Then, the resultant is subjected to filtration using a pressurized filtering-machine with a 5 µm membrane filter, followed by observing the residue on the membrane filter. When no solid product is observed at the time, it is determined that the liquid crystal polyester powder is soluble in an aprotic solvent.

In the present embodiment, the particle size of the liquid crystal polyester powder is measured by a dry sieving test method in accordance with JIS K 0069 (1992). Specifically, the particle size is determined by subjecting the liquid crystal polyester powder to a classification operation using sieves having a mesh size of 63 µm, 106 µm, 250 µm, 425 µm, 710 µm, 1000 µm, 1180 µm, 1700 µm, 2000 µm, 2360 µm, 2800 µm and 3350 µm.

The phrase "proportion of particles having a particle size of less than 250 µm" means the proportion (% by mass) of the amount of powder passing through a sieve having a mesh size of 250 µm relative to the total amount of the entire powder (100% by mass).

The phrase "proportion of particles having a particle size of less than 106 µm" means the proportion (% by mass) of the amount of powder passing through a sieve having a mesh size of 106 µm relative to the total amount of the entire powder (100% by mass).

The phrase "proportion of particles having a particle size of less than 425 µm" means the proportion (% by mass) of the amount of powder passing through a sieve having a mesh size of 425 µm relative to the total amount of the entire powder (100% by mass).

The term "center particle size (D50)" means the median diameter.

In the liquid crystal polyester powder according to the present embodiment, the proportion of particles having a particle size of less than 250 µm is 23.5% by mass or less, preferably 23.0% by mass or less, more preferably 20.0% by mass or less, even more preferably 15.0% by mass or less, particularly preferably 14.5% by mass or less, and most preferably 14.0% by mass or less, the particle size being measured by the dry sieving test method.

In the liquid crystal polyester powder according to the present embodiment, the proportion of particles having a particle size of less than 250 µm is preferably 10.0% by mass or more, more preferably 11.0% by mass or more, even more preferably 11.5% by mass or more, particularly preferably 11.8% by mass or more, and most preferably 12.0% by mass or more, the particle size being measured by the dry sieving test method.

When the proportion of particles having a particle size of less than 250 µm is equal to or less than the upper limit of the above-mentioned range, the change in the solution viscosity for the flow starting temperature of the liquid crystal polyester powder is suppressed. In contrast, when the proportion of particles having a particle size of less than 250 µm is equal to or more than the lower limit of the above-mentioned preferable range, an excessive decrease in the solution viscosity of a solution in which the liquid crystal polyester powder is dissolved in an aprotic solvent is prevented, and therefore the solution viscosity is easily adjusted to a predetermined value.

In the liquid crystal polyester powder according to the present embodiment, the proportion of particles having a particle size of less than 250 µm may be, for example, 10.0% by mass to 23.5% by mass, 10.5% by mass to 23.0% by mass, 11.0% by mass to 22.5% by mass, 11.5% by mass to 15.0% by mass, 11.8% by mass to 14.5% by mass, or 12.0% by mass to 14.0% by mass, the particle size being measured by the dry sieving test method.

In the liquid crystal polyester powder according to the present embodiment, the proportion of particles having a particle size of less than 106 µm is preferably 10.0% by mass or less, more preferably 7.0% by mass or less, and even more preferably 5.0% by mass or less, the particle size being measured by the dry sieving test method.

In the liquid crystal polyester powder according to the present embodiment, the proportion of particles having a particle size of less than 106 µm is preferably 1.5% by mass or more, more preferably 2.0% by mass or more, and even more preferably 3.0% by mass or more, the particle size being measured by the dry sieving test method.

When the proportion of particles having a particle size of less than 106 µm is equal to or less than the upper limit of the above-mentioned preferable range, the change in the solution viscosity for the flow starting temperature of the liquid crystal polyester powder is easily suppressed. In contrast, when the proportion of particles having a particle size of less than 106 µm is equal to or more than the lower limit of the above-mentioned preferable range, an excessive decrease in the solution viscosity of a solution in which the liquid crystal polyester powder is dissolved in an aprotic solvent is prevented, and therefore the solution viscosity is easily adjusted to a predetermined value.

In the liquid crystal polyester powder according to the present embodiment, the proportion of particles having a particle size of less than 106 µm may be, for example, 1.5% by mass to 10.0% by mass, 2.0% by mass to 7.0% by mass, or 3.0% by mass to 5.0% by mass, the particle size being measured by the dry sieving test method.

In the liquid crystal polyester powder according to the present embodiment, the proportion of particles having a particle size of less than 425 µm is preferably 50.0% by mass or less, more preferably 30.0% by mass or less, even more preferably 20.0% by mass or less, and particularly preferably 15.0% by mass or less, the particle size being measured by the dry sieving test method.

In the liquid crystal polyester powder according to the present embodiment, the proportion of particles having a particle size of less than 425 µm is preferably 5.0% by mass or more, more preferably 7.5% by mass or more, even more preferably 10.0% by mass or more, and particularly preferably 12.5% by mass or more, the particle size being measured by the dry sieving test method.

When the proportion of particles having a particle size of less than 425 µm is equal to or less than the upper limit of the above-mentioned preferable range, the change in the solution viscosity for the flow starting temperature of the liquid crystal polyester powder is easily suppressed. In contrast, when the proportion of particles having a particle size of less than 425 µm is equal to or more than the lower limit of the above-mentioned preferable range, an excessive decrease in the solution viscosity of a solution in which the liquid crystal polyester powder is dissolved in an aprotic solvent is prevented, and therefore the solution viscosity is easily adjusted to a predetermined value.

In the liquid crystal polyester powder according to the present embodiment, the proportion of particles having a particle size of less than 425 µm may be, for example, 5.0% by mass to 50.0% by mass, 7.5% by mass to 40.0% by mass, 10.0% by mass to 30.0% by mass, 10.0% by mass to 20.0% by mass, 10.0% by mass to 15.0% by mass, or 12.5% by mass to 15.0% by mass, the particle size being measured by the dry sieving test method.

In the liquid crystal polyester powder according to the present embodiment, the center particle size (D50) measured by the dry sieving test method is preferably 650 µm to 1200 µm, more preferably 700 µm to 1150 µm, even more preferably 750 µm to 1100 µm, and particularly preferably 800 µm to 1100 µm.

When the center particle size (D50) is equal to or less than the upper limit of the above-mentioned preferable range, an excessive decrease in the solution viscosity of a solution in which the liquid crystal polyester powder is dissolved in an aprotic solvent is prevented, and therefore the solution viscosity is easily adjusted to a predetermined value. In contrast, when the center particle size (D50) is equal to or more than the lower limit of the above-mentioned preferable range, the change in the solution viscosity for the flow starting temperature of the liquid crystal polyester powder is easily suppressed.

In the present embodiment, when an impact mill is used as a pulverizer on pulverization after melt-polymerization or solid phase polymerization, examples of a method for controlling the particle size of the liquid crystal polyester powder include a method in which a rotation number of a mill blade and/or a screen to be used is changed. When a jet mill is used as a pulverizer, examples of a method for controlling the particle size include a method in which a rotation speed of a classification-rotor, a pressure at a mill-nozzle, and/or a processing speed is changed.

Examples of the liquid crystal polyester powder according to the present embodiment include one containing a liquid crystal polyester having a structural unit (u1) of the following formula (1), a structural unit (u2) of the following formula (2) and a structural unit (u3) of the following formula (3).

-O-Ar¹-CO- (1)

-CO-Ar²-CO- (2)

-X- Ar³-Y- (3)

In the formulae (1) to (3), Ar¹ is a 1,4-phenylene group, a 2,6-naphthalenediyl group or a 4,4'-biphenylylene group. Ar² is a 1,4-phenylene group, a 1,3-phenylene group or a 2,6-naphthalenediyl group. Ar³ is a 1,4-phenylene group or a 1,3-phenylene group. X is -NH-. Y is -O- or -NH-. Any of the hydrogen atoms in Ar¹, Ar² or Ar³ may be each independently substituted with a halogen atom, an alkyl group having a carbon number of 1 to 10 or an aryl group having a carbon number of 6 to 20.

Examples of the halogen atom in Ar¹, Ar² or Ar³ include a fluorine atom, a chlorine atom, a bromine atom and an iodine atom.

Examples of the alkyl group having a carbon number of 1 to 10 in Ar¹, Ar² or Ar³ include a methyl group, an ethyl group, a n-propyl group, an isopropyl group, a n-butyl group, an isobutyl group, a s-butyl group, a t-butyl group, a n-hexyl group, a 2-ethylhexyl group, a n-octyl group and a n-decyl group.

Examples of the aryl group having a carbon number of 6 to 20 in Ar¹, Ar² or Ar³ include a phenyl group, an o-tolyl group, a m-tolyl group, a p-tolyl group, a 1-naphthyl group and a 2-naphthyl group.

When the hydrogen atoms in Ar¹, Ar² or Ar³ are substituted with these groups, the number thereof per group of Ar¹, Ar² or Ar³ is each independently 2 or less generally, and preferably 1 or less.

The structural unit (u1) is a structural unit derived from an aromatic hydroxycarboxylic acid, the structural unit (u2) is a structural unit derived from an aromatic dicarboxylic acid, and the structural unit (u3) is a structural unit derived from an aromatic diamine or an aromatic amine having a phenolic hydroxyl group.

The liquid crystal polyester contained in the liquid crystal polyester powder according to the present embodiment may have, instead of the structural units (u1) to (u3), an ester-forming derivative or an amide-forming derivative of the structural units.

The term "derived from" means that the chemical structure changes due to polymerization.

In the present embodiment, it is preferable that Ar¹ be a 2,6-naphthalenediyl group, Ar² be a 1,3-phenylene group, Ar³ be a 1,4-phenylene group, and Y be -O-.

Examples of the ester-forming derivative of a carboxy group include: high-reactive derivatives in which a carboxy group promotes a reaction to produce a polyester, such as acid chlorides and acid anhydrides; and derivatives in which a carboxy group forms an ester together with an alcohol or an ethylene glycol which produces a polyester by transesterification.

Examples of the ester-forming derivative of a phenolic hydroxyl group include derivatives in which a phenolic hydroxyl group is made into an ester together with a carboxylic acid.

Examples of the amide-forming derivative of an amino group include derivatives in which an amino group is made into an amide together with a carboxylic acid.

Although examples of the repeating structural unit of the liquid crystal polyester in the present embodiment are shown below, the repeating structural unit is not limited to these examples.

Examples of the structural unit (u1) of formula (1) include structural units derived from p-hydroxybenzoic acid, 6-hydroxy-2-naphthoic acid or 4'-hydroxy-4-biphenylcarboxylic acid, and two or more of the above-mentioned structural units may be included in all structural units. A liquid crystal polyester including a structural unit derived from 6-hydroxy-2-naphthoic acid is preferable among these structural units.

The amount of the structural unit (u1) relative to the total amount (100% by mol) of all structural units constituting the liquid crystal polyester is preferably 30% by mol to 80% by mol, more preferably 40% by mol to 70% by mol, and even more preferably 45% by mol to 65% by mol.

When the amount of the structural unit (u1) is within the above-mentioned range, the solubility in a solvent is favorable, and the liquid crystallinity is easily exhibited.

Examples of the structural unit (u2) of formula (2) include structural units derived from terephthalic acid, isophthalic acid or 2,6-naphthalenedicarboxylic acid, and two or more of the above-mentioned structural units may be included in all structural units. A liquid crystal polyester having a structural unit derived from isophthalic acid is preferably used from the viewpoint of the solubility in a solvent among these structural units.

The amount of the structural unit (u2) relative to the total amount (100% by mol) of all structural units constituting the liquid crystal polyester is preferably 10% by mol to 35% by mol, more preferably 15% by mol to 30% by mol, and even more preferably 17.5% by mol to 27.5% by mol.

When the amount of the structural unit (u2) is within the above-mentioned range, the liquid crystallinity is favorable, and the solubility in a solvent is also favorable.

Examples of the structural unit (u3) of formula (3) include structural units derived from 3-aminophenol, 4-aminophenol, 1,4-phenylenediamine or 1,3-phenylenediamine, and two or more of the above-mentioned structural units may be included in all structural units. A liquid crystal polyester having a structural unit derived from 4-aminophenol is preferably used from the viewpoint of the reactivity among these structural units.

The amount of the structural unit (u3) relative to the total amount (100% by mol) of all structural units constituting the liquid crystal polyester is preferably 10% by mol to 35% by mol, more preferably 15% by mol to 30% by mol, and even more preferably 17.5% by mol to 27.5% by mol.

When the amount of the structural unit (u3) is within the above-mentioned range, the liquid crystallinity is favorable, and the solubility in a solvent is also favorable.

Although the structural unit (u3) is preferably used in substantially the same amount as the structural unit (u2), the polymerization degree of the liquid crystal polyester can be controlled by adjusting the amount of the structural unit (u3) to -10% by mol to +10% by mol relative to the amount of the structural unit (u2).

However, the total amount of the structural unit (1), the structural unit (2) and the structural unit (3) constituting the liquid crystal polyester does not exceed 100% by mol.

In the liquid crystal polyester powder according to the present embodiment, the amount of the liquid crystal polyester relative to the total amount (100% by mass) of the liquid crystal polyester powder may be 90% by mass or more, 95% by mass or more, 99% by mass or more, or 100% by mass, for example.

### <Method for producing liquid crystal polyester powder>

Although the method for producing the liquid crystal polyester powder according to the present embodiment is not particularly limited, examples thereof include a method in which an aromatic hydroxy acid corresponding to the structural unit (u1), an acylated product obtained by acylating a phenolic hydroxyl group or an amino group of an aromatic amine or an aromatic diamine having a phenolic hydroxyl group corresponding to the structural unit (u3) with an excessive amount of fatty acid anhydride, and an aromatic dicarboxylic acid corresponding to the structural unit (u2) are subjected to melt-polymerization by transesterification and transamidation (polycondensation), followed by subjecting the resultant to solid phase polymerization.

The addition amount of the fatty acid anhydride in the acylation relative to the total amount of the phenolic hydroxyl group and the amino group is preferably 1.0-fold equivalent to 1.2-fold equivalent, and more preferably 1.05-fold equivalent to 1.10-fold equivalent. When the addition amount of the fatty acid anhydride is within the above-mentioned range, the reactivity of the acylated product or feedstock monomers is favorable at the time of the transesterification and transamidation (polycondensation), and the resultant liquid crystal polyester is not excessively colored.

The acylation is preferably conducted at 130°C to 180°C for 5 minutes to 10 hours, and more preferably at 140°C to 160°C for 10 minutes to 3 hours.

Although the fatty acid anhydride available in acylation is not particularly limited, examples thereof include anhydrous acetic acid, anhydrous propionic acid, anhydrous butyric acid, anhydrous isobutyric acid, anhydrous valeric acid, anhydrous pivalic acid, anhydrous 2-ethylhexanoic acid, anhydrous monochloroacetic acid, anhydrous dichloroacetic acid, anhydrous trichloroacetic acid, anhydrous monobromoacetic acid, anhydrous dibromoacetic acid, anhydrous tribromoacetic acid, anhydrous monofluoroacetic acid, anhydrous difluoroacetic acid, anhydrous trifluoroacetic acid, anhydrous glutaric acid, anhydrous maleic acid, anhydrous succinic acid, and anhydrous β-bromopropionic acid, and two or more thereof may be mixed to be used. In the present embodiment, anhydrous acetic acid, anhydrous propionic acid, anhydrous butyric acid, or anhydrous isobutyric acid is preferable, and anhydrous acetic acid is more preferable.

In the transesterification and transamidation (polycondensation), it is preferable that the content of an acyl group in an acylated product be 0.8-fold equivalent to 1.2-fold equivalent relative to the content of a carboxyl group.

It is preferable that the transesterification and transamidation (polycondensation) be conducted by raising the temperature to 400°C at a rate of 0.1°C/minute to 50°C/minute. It is more preferable that the transesterification and transamidation (polycondensation) be conducted by raising the temperature to 350°C at a rate of 0.3°C/minute to 5°C/minute. It is even more preferable that the transesterification and transamidation (polycondensation) be conducted by raising the temperature to 300°C at a rate of 0.3°C/minute to 5°C/minute.

When an acylated product and a carboxylic acid are subjected to transesterification and transamidation (polycondensation), it is preferable that a fatty acid produced as a by-product and an unreacted fatty acid anhydride be distilled away from the system by evaporating or the like.

The acylation reaction and the transesterification and transamidation (polycondensation) may be conducted in the presence of a catalyst. As the catalyst, a catalyst conventionally used to conduct polymerization of polyester may be used, and examples thereof include: metallic salt catalysts such as magnesium acetate, stannous acetate, tetrabutyl titanate, lead acetate, sodium acetate, potassium acetate, and antimony trioxide; and organic compound catalysts such as N,N-dimethylaminopyridine, and N-methylimidazole.

Among these catalysts, heterocyclic compounds containing at least two nitrogen atoms such as N, N-dimethylaminopyridine and N-methylimidazole are preferably used (see Japanese Patent Application Publication No. 2002-146003).

The catalyst is usually added at the time of supplying monomers, and it is not always necessary to remove the catalyst even after acylation. When the catalyst is not removed, transesterification can be carried out directly.

After polycondensation by transesterification and transamidation described above, the resultant liquid crystal polyester may be pelletized and molded by a known method. Furthermore, the resultant liquid crystal polyester may be pulverized by a known method.

The flow starting temperature of the liquid crystal polyester obtained by polycondensation by transesterification and transamidation described above is preferably 170°C to 220°C, and more preferably 180°C to 210°C, for example.

In the present embodiment, each flow starting temperature of the liquid crystal polyester powder and the liquid crystal polyester is determined by filling a cylinder equipped with a die having a nozzle having an inner diameter of 1 mm and a length of 10 mm with the liquid crystal polyester powder or the liquid crystal polyester, followed by melting the liquid crystal polyester while raising the temperature thereof at a rate of 4°C/min under a load of 9.8 MPa (100 kg/cm²) to extrude the resultant from the nozzle, and measuring the temperature when the melt-viscosity reaches 4800 Pa·s (48000 P).

The solid phase polymerization is preferably conducted by a known solid phase polymerization method after extracting polymers from the melt-polymerization step and then pulverizing the resultant into a powder-form or a flake-form.

Specifically, a method in which a heating treatment is conducted in a solid state under an inert atmosphere such as a nitrogen atmosphere, preferably at 200°C to 300°C for 1 hour to 20 hours, more preferably at 240°C to 280°C for 2 hours to 10 hours, particularly preferably at 250°C to 270°C for 4 hours to 6 hours, may be adopted.

The solid phase polymerization may be conducted under stirring, or may be conducted in a still state without stirring. A melt-polymerization tank and a solid phase polymerization tank may employ the same reaction tank by providing an appropriate stirring mechanism.

After the solid phase polymerization, the resultant liquid crystal polyester powder is pulverized by a known method to adjust the particle size thereof.

The flow starting temperature of the thus obtained liquid crystal polyester powder is preferably, for example, 290°C to 315°C, more preferably 290°C to 310°C, and even more preferably 295°C to 305°C.

When the flow starting temperature of the liquid crystal polyester powder is equal to or more than the lower limit of the above-mentioned preferable range, the heat resistance of the resultant molded product tends to be further improved. In contrast, when the flow starting temperature of the liquid crystal polyester powder is equal to or less than the upper limit of the above-mentioned preferable range, an excessive increase in melting temperature is suppressed, and the handleability is further improved.

The method for producing the liquid crystal polyester powder described above may be conducted by using a batch-wise device, a continuous device, or the like, for example.

### (Method for producing the liquid crystal polyester solution composition)

A method for producing a liquid crystal polyester solution composition of the present embodiment is a production method in which the liquid crystal polyester powder of the above-mentioned embodiment is dissolved in an aprotic solvent to obtain the liquid crystal polyester solution composition.

In the present embodiment, the term "aprotic solvent" means a solvent including an aprotic compound.

Examples of the aprotic solvent include halogen-based solvents such as 1-chlorobutane, chlorobenzene, 1,1-dichloroethane, 1,2-dichloroethane, chloroform, and 1,1,2,2-tetrachloroethane; ether-based solvents such as diethyl ether and tetrahydrofuran, and 1,4-dioxane; ketone-based solvents such as acetone and cyclohexanone; ester-based solvents such as ethyl acetate; lactone-based solvents such as γ-butyrolactone; carbonate-based solvents such as ethylene carbonate and propylene carbonate; amine-based solvents such as triethylamine and pyridine; nitrile-based solvents such as acetonitrile and succinonitrile; amide-based solvents such as N,N'-dimethylformamide, N,N'-dimethylacetamide, tetramethylurea and N-methylpyrrolidone; nitro-based solvents such as nitromethane and nitrobenzene; sulfide-based solvents such as dimethyl sulfoxide and sulfolane; and phosphoric acid-based solvents such as hexamethylphosphoramide and tri-n-butyl phosphate.

Among these, a solvent free from any halogen atoms is preferably used from the viewpoint of environmental influence, and a solvent having a dipole moment of 3 to 5 is preferably used from the viewpoint of solubility.

Specifically, an amide-based solvent such as N,N'-dimethylformamide, N,N'-dimethylacetamide, tetramethylurea, or N-methylpyrrolidone, or a lactone-based solvent such as γ-butyrolactone is preferably used, and N,N'-dimethylformamide, N,N'-dimethylacetamide, or N-methylpyrrolidone is more preferably used.

In the liquid crystal polyester solution composition prepared by the production method of the present embodiment, the amount of the liquid crystal polyester powder relative to the total amount (100% by mass) of the liquid crystal polyester solution composition is preferably 1% by mass to 20% by mass, and more preferably 5% by mass to 10% by mass, for example.

In the liquid crystal polyester solution composition prepared by the production method of the present embodiment, the amount of the aprotic solvent relative to the total amount (100% by mass) of the liquid crystal polyester solution composition is preferably 80% by mass to 99% by mass, and more preferably 90% by mass to 95% by mass, for example.

In the method for producing the liquid crystal polyester solution composition of the present embodiment, components other than the liquid crystal polyester powder and the aprotic solvent may be formulated, as needed.

In the method for producing the liquid crystal polyester solution composition of the present embodiment, the formulation order of each component is not particularly limited, and the liquid crystal polyester powder and the other components to be formulated as needed may be mixed with the aprotic solvent at once or in an appropriate order to prepare the liquid crystal polyester solution composition, for example.

The solution viscosity of the liquid crystal polyester solution composition of the present embodiment is appropriately set depending on the intended use thereof, and is set at 400 mPa·s to 7000 mPa·s, preferably 800 mPa·s to 6000 mPa·s, more preferably 1000 mPa·s to 5000 mPa·s, and particularly preferably 1000 mPa·s to 3000 mPa·s, for example.

In the present embodiment, the solution viscosity of the liquid crystal polyester solution composition is measured using a B-type viscometer under the following measurement conditions.

Measurement conditions: Temperature is set at 23°C, and the rotational number of a rotor is set at 20 rpm.

The liquid crystal polyester powder according to the present invention encompasses the following aspects.
<1> A liquid crystal polyester powder soluble in an aprotic solvent, wherein
   the proportion of particles having a particle size of less than 250 µm is 10.5% by mass to 23.5% by mass, the particle size being measured by a dry sieving test method in accordance with JIS K 0069 (1992), and
   the center particle size (D50) measured by the dry sieving test method is 650 µm to 1400 µm.
<2> A liquid crystal polyester powder soluble in an aprotic solvent, wherein
   the proportion of particles having a particle size of less than 250 µm is 11.5% by mass to 23.5% by mass or less, the particle size being measured by a dry sieving test method in accordance with JIS K 0069 (1992), and
   the center particle size (D50) measured by the dry sieving test method is 650 µm to 1200 µm.
<3> A liquid crystal polyester powder soluble in an aprotic solvent, wherein
   the proportion of particles having a particle size of less than 250 µm is 11.5% by mass to 14.0% by mass or less, the particle size being measured by a dry sieving test method in accordance with JIS K 0069 (1992), and
   the center particle size (D50) measured by the dry sieving test method is 800 µm to 1150 µm.
<4> The liquid crystal polyester powder according to any one of <1> to <3> described above, wherein the proportion of particles having a particle size of less than 106 µm is 0.6% by mass to 7.0% by mass, preferably 2.0% by mass to 7.0% by mass, and more preferably 2.0% by mass to 5.0% by mass, the particle size being measured by the dry sieving test method.
<5> The liquid crystal polyester powder according to any one of <1> to <4> described above, wherein the proportion of particles having a particle size of less than 425 µm is 10.0% by mass to 40.0% by mass, preferably 10.0% by mass to 30.0% by mass, and more preferably 12.0% by mass to 16.0% by mass, the particle size being measured by the dry sieving test method.
<6> The liquid crystal polyester powder according to any one of <1> to <5> described above, including a liquid crystal polyester having a structural unit (u1) of the following formula (1), a structural unit (u2) of the following formula (2) and a structural unit (u3) of the following formula (3), wherein
   the amount of the structural unit (u1) is 30% by mol to 80% by mol, the amount of the structural unit (u2) is 10% by mol to 35% by mol, and the amount of the structural unit (u3) is 10% by mol to 35% by mol, relative to the total structural units constituting the liquid crystal polyester.

   -O-Ar¹-CO- (1)

   -CO-Ar²-CO- (2)

   -X- Ar³-Y- (3)

   In the formulae (1) to (3), Ar¹ is a 1,4-phenylene group, a 2,6-naphthalenediyl group or a 4,4'-biphenylylene group. Ar² is a 1,4-phenylene group, a 1,3-phenylene group or a 2,6-naphthalenediyl group. Ar³ is a 1,4-phenylene group or a 1,3-phenylene group. X is -NH-. Y is -O- or -NH-. Any of the hydrogen atoms in Ar¹, Ar² or Ar³ may be each independently substituted with a halogen atom, an alkyl group having a carbon number of 1 to 10 or an aryl group having a carbon number of 6 to 20.
<7> The liquid crystal polyester powder according to <6> described above, wherein the structural unit (u1) is at least one structural unit selected from the group consisting of a structural unit derived from p-hydroxybenzoic acid, a structural unit derived from 6-hydroxy-2-naphthoic acid, and a structural unit derived from 4'-hydroxy-4-biphenylcarboxylic acid.
<8> The liquid crystal polyester powder according to <6> or <7> described above, wherein the structural unit (u2) is at least one structural unit selected from the group consisting of a structural unit derived from terephthalic acid, a structural unit derived from isophthalic acid, and a structural unit derived from 2,6-naphthalenedicarboxylic acid.
<9> The liquid crystal polyester powder according to any one of <6> to <8> described above, wherein the structural unit (u3) is at least one structural unit selected from the group consisting of a structural unit derived from 3-aminophenol, a structural unit derived from 4-aminophenol, a structural unit derived from 1,4-phenylenediamine, and a structural unit derived from 1,3-phenylenediamine.
   The method for producing the liquid crystal polyester solution composition according to the present invention encompasses the following aspects.
<10> A method for producing the liquid crystal polyester solution composition, including dissolving the liquid crystal polyester powder of any one of <1> to <9> described above in an aprotic solvent to obtain the liquid crystal polyester solution composition.
<11> The method for producing the liquid crystal polyester solution composition according to <10> described above, wherein the aprotic solvent is at least one solvent selected from the group consisting of N,N'-dimethylformamide, N,N'-dimethylacetamide, tetramethylurea, N-methylpyrrolidone and γ-butyrolactone.
<12> The method for producing the liquid crystal polyester solution composition according to <10> or <11> described above, wherein
   the amount of the liquid crystal polyester powder relative to the total amount (100% by mass) of the liquid crystal polyester solution composition is 1% by mass to 20% by mass, and preferably 5% by mass to 10% by mass, and
   the amount of the aprotic solvent relative to the total amount (100% by mass) of the liquid crystal polyester solution composition is 99% by mass to 80% by mass, and preferably 95% by mass to 90% by mass.
<13> The method for producing the liquid crystal polyester solution composition according to any one of <10> to <12> described above, wherein the solution viscosity of the liquid crystal polyester solution composition at 23°C is 400 mPa·s to 7000 mPa·s, preferably 800 mPa·s to 6000 mPa·s, more preferably 1000 mPa·s to 5000 mPa·s, and even more preferably 1000 mPa·s to 3000 mPa·s.

The liquid crystal polyester powder of the embodiment described above makes it possible to prepare a liquid crystal polyester solution composition in which the change in the solution viscosity for the flow starting temperature of the liquid crystal polyester powder is suppressed.

The liquid crystal polyester powder of the present embodiment is soluble in an aprotic solvent. The particle size of a solute is generally made as small as possible in order to increase the solubility of the solute in the solvent. In contrast, particles (fine powder) having a specific particle size are reduced to a specific proportion in the liquid crystal polyester powder of the embodiment. However, the conventional problem, that is, the problem in which the solution viscosity of the liquid crystal polyester solution composition changes significantly when a liquid crystal polyester powder having a higher flow starting temperature is used, and the controllability of the solution viscosity becomes poor can be solved by adopting the liquid crystal polyester powder according to the present embodiment.

Furthermore, the adoption of the liquid crystal polyester powder of the present embodiment makes it possible to increase the adhesion strength with a metal foil and the mechanical strength when made into a film.

The liquid crystal polyester solution composition of the present embodiment is useful as a non-anisotropic liquid crystal polymer film or an insulating material for a multilayer printed circuit board or a flexible printed wiring board (such as an insulating material for a flexible copper-clad laminate).

### EXAMPLES

Hereinafter, the present invention will be explained in further detail with reference to examples. However, the present invention is not limited to the examples described below.

### [Measurement of flow starting temperature of liquid crystal polyester powder]

A cylinder equipped with a die having a nozzle having an inner diameter of 1 mm and a length of 10 mm was filled with approximately 2 g of a liquid crystal polyester powder using a flow tester (manufactured by Shimadzu Corporation under the model number of "CFT-500"). Then, the liquid crystal polyester powder was melted and extruded from the nozzle while raising the temperature at a rate of 4°C/min under a load of 9.8 MPa (100 kg/cm²) to measure the temperature at which the melt-viscosity reached 4800 Pa·s (48000 P) (flow starting temperature).

### [Measurement of particle size of the liquid crystal polyester powder]

The particle size of the liquid crystal polyester powder was measured by a dry sieving test method in accordance with JIS K 0069 (1992).

Specifically, the particle size of the liquid crystal polyester powder was measured using a low-tap type shaker (manufactured by KYOEISHA CHEMICAL Co., LTD.) with sieves having a mesh size of 63 µm, 106 µm, 250 µm, 425 µm, 710 µm, 1000 µm, 1180 µm, 1700 µm, 2000 µm, 2360 µm, 2800 µm and 3350 µm.

### [Measurement of solution viscosity of liquid crystal polyester solution]

The solution viscosity of a liquid crystal polyester solution was measured using a B-type viscometer (manufactured by TOKI SANGYO CO., LTD., under the model number of "TV-22") under the following measurement conditions.

Measurement conditions: Temperature was set at 23°C, and the rotational number of rotor was set at 20 rpm.

### <Preparation Examples of liquid crystal polyester powders (A1) to (A5)>

### (Examples 1 to 4, and Comparative Example 1)

940.9 g (5.0 mol) of 6-hydroxy-2-naphthoic acid, 377.9 g (2.5 mol) of acetaminophen, 415.3 g (2.5 mol) of isophthalic acid, and 867.8 g (8.4 mol) of anhydrous acetic acid were put in a reactor equipped with a stirrer, a torque meter, a nitrogen gas inlet tube, a thermometer and a reflux condenser, and the gas in the reactor was replaced with nitrogen gas, followed by raising the temperature therein from room temperature to 140°C over 60 minutes while stirring the mixture under a nitrogen gas stream to reflux the mixture at 140°C for 3 hour. Then, the temperature was raised from 140°C to 300°C over 5 hours while distilling off acetic acid produced as a by-product and unreacted anhydrous acetic acid, followed by maintaining the temperature at 300°C for 30 minutes, and then the content was taken out from the reactor to cool to room temperature, as a result of which a solid product was obtained.

The resultant solid product was pulverized with a pulverizer manufactured by Orient Co., Ltd., (model number: VM-16, rotational number: 1500 rpm) to obtain a powdered liquid crystal polyester (A0). The flow starting temperature of this liquid crystal polyester (A0) was 193.3°C.

The liquid crystal polyester (A0) was heated from room temperature to 160°C over 2 hours and 20 minutes under a nitrogen atmosphere, heated from 160°C to 180°C over 3 hours and 20 minutes, and then maintained at 180°C for 5 hours to allow solid phase polymerization to proceed.

After the solid phase polymerization, the resultant was cooled. Then, the resultant was pulverized using a pulverizer manufactured by Orient Co., Ltd., (model number: VM-16, rotational number: 1500 rpm) with a punching metal screen (pulverizing screen) having each size shown in Table 1 to obtain liquid crystal polyester powders (A1) to (A5). The flow starting temperature of these liquid crystal polyester powders (A1) to (A5) was 220°C.

The median diameter (D50) of each of the resultant liquid crystal polyester powders (A1) to (A5) was measured as the center particle size, and the results thereof are shown in Table 1.

The proportion of particles having a particle size of less than 425 µm which passed through a sieve having a mesh size of 425 µm, the proportion of particles having a particle size less than 250 µm which passed through a sieve having a mesh size of 250 µm, and the proportion of particles having a particle size less than 106 µm which passed through a sieve having a mesh size of 106 µm are shown in Table 1.

**Table 1**

| | | Example 1 | Example 2 | Comparative Example 1 | Example 3 | Example 4 |
|---|---|---|---|---|---|---|
| Pulverizing screen | | 3 mm screen | 5 mm screen | 2 mm screen | 6 mm screen | 4 mm screen |
| Liquid crystal polyester powder | | (A1) | (A2) | (A3) | (A4) | (A5) |
| Center particle size (D50) | µm | 763 | 1060 | 371 | 1320 | 988 |
| Proportion of particles having a particle size of less than 425 µm | % by mass | 26.9 | 14.8 | 53.7 | 16.9 | 24.1 |
| Proportion of particles having a particle size of less than 250 µm | % by mass | 22.9 | 12.3 | 39.2 | 11.1 | 14.8 |
| Proportion of particles having a particle size of less than 106 µm | % by mass | 3.3 | 3.3 | 19.9 | 1.3 | 0.9 |

The liquid crystal polyester powders (A1) to (A5) were each heated from room temperature to 180°C over 1 hour and 25 minutes under a nitrogen atmosphere.

Then, the temperature thereof was raised from 180°C to each reaction temperature shown in Table 2 at a rate of 0.2°C/min, and then maintained at each reaction temperature for 5 hours to allow solid phase polymerization to further proceed.

After the additional solid phase polymerization, each resultant was cooled to obtain each liquid crystal polyester powder. The flow starting temperature of each liquid crystal polyester powder obtained by solid phase polymerization at each reaction temperature is shown in Table 2.

### <Preparation of liquid crystal polyester solution>

8 parts by mass of each resultant liquid crystal polyester powder was added to 92 parts by mass of N-methylpyrrolidone (having a boiling point (at 1 atm) of 204°C), followed by stirring the mixture under a nitrogen atmosphere at 140°C for 4 hours to obtain each liquid crystal polyester solution.

Each resultant liquid crystal polyester powder was dissolved in N-methylpyrrolidone. The solution viscosity of each liquid crystal polyester solution was measured and the resultants thereof are shown in Table 2.

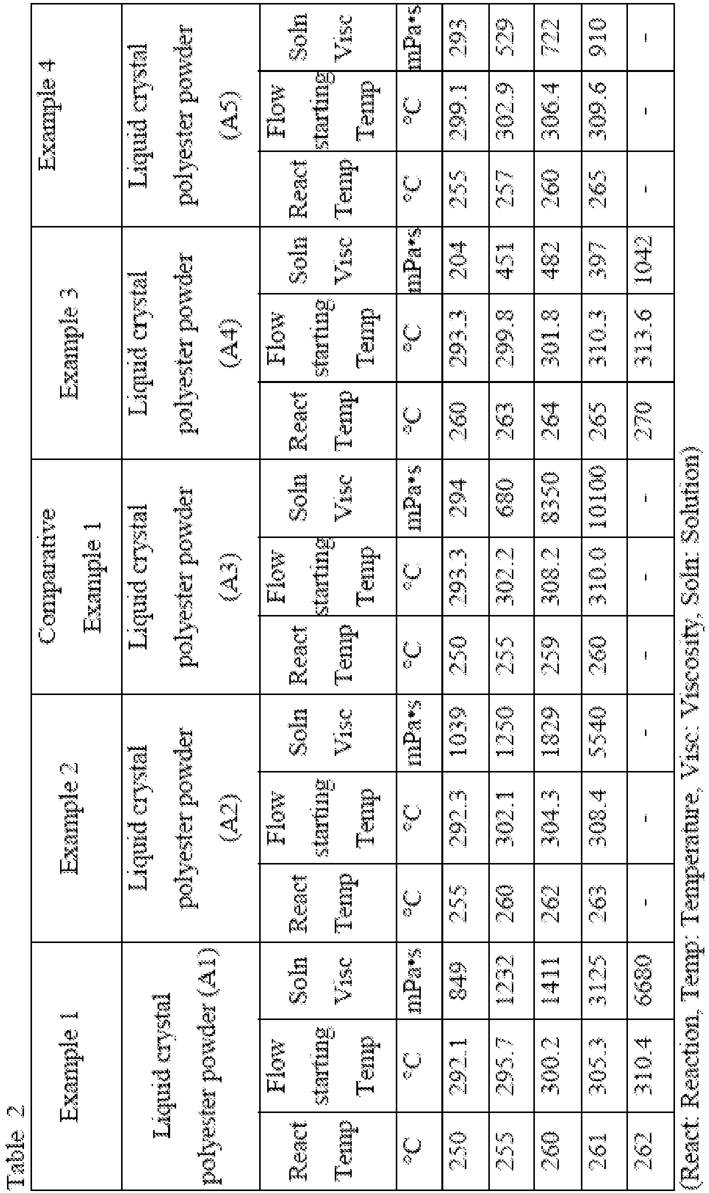

### [Change in solution viscosity for flow starting temperature in liquid crystal polyester powder]

The change in solution viscosity for flow starting temperature of the liquid crystal polyester powder of each example was evaluated based on the results shown in Table 2.

Specifically, a semi-logarithmic graph was drawn with the horizontal axis representing the flow starting temperature (°C) and the vertical axis representing the logarithmic value of the solution viscosity (mPa·s), and an equation of the regression line was obtained. The slope of the equation of the regression line obtained in the liquid crystal polyester powder of each example is shown below.

The smaller the value of this slope is, the smaller the rate of change in solution viscosity is.

Example 1:0.1106
Example 2: 0.0897
Comparative Example 1: 0.2247
Example 3: 0.0355
Example 4: 0.1067

It was confirmed that the liquid crystal polyester powders of Examples 1 to 4 according to the present invention suppress a change in the solution viscosity for the flow starting temperature of the liquid crystal polyester powders as compared with the liquid crystal polyester powder of Comparative Example 1. Namely, the solution viscosity of the liquid crystal polyester powders of Examples 1 to 4 is easily adjusted to a desired value, and the liquid crystal polyester powders have excellent controllability of the solution viscosity.

In addition, the solution viscosity of the liquid crystal polyester powders of Examples 1 and 2 is easily adjusted to about 1000 mPa·s to 3000 mPa·s in an flow starting temperature range of 295°C to 305°C.

## Claims

1. A liquid crystal polyester powder soluble in an aprotic solvent, wherein a proportion of particles having a particle size of less than 250 µm is 23.5% by mass or less, the particle size being measured by a dry sieving test method in accordance with JIS K 0069 (1992).

2. The liquid crystal polyester powder according to claim 1, wherein a center particle size (D50) measured by the dry sieving test method is 650 µm to 1200 µm.

3. The liquid crystal polyester powder according to claim 1 or 2, wherein the proportion of particles having a particle size of less than 250 µm is 11.5% by mass or more, the particle size being measured by the dry sieving test method.

4. The liquid crystal polyester powder according to any one of claims 1 to 3, wherein a proportion of particles having a particle size of less than 106 µm is 10.0% by mass or less, the particle size being measured by the dry sieving test method.

5. The liquid crystal polyester powder according to any one of claims 1 to 4, wherein a proportion of particles having a particle size of less than 106 µm is 1.5% by mass or more, the particle size being measured by the dry sieving test method.

6. The liquid crystal polyester powder according to any one of claims 1 to 5, comprising a liquid crystal polyester having a structural unit (u1) of formula (1), a structural unit (u2) of formula (2) and a structural unit (u3) of formula (3), and
an amount of the structural unit (u1) is 30% by mol to 80% by mol, an amount of the structural unit (u2) is 10% by mol to 35% by mol, and an amount of the structural unit (u3) is 10% by mol to 35% by mol, relative to total structural units constituting the liquid crystal polyester,
-O-Ar¹-CO- (1)
-CO-Ar²-CO- (2)
-X- Ar³-Y- (3)
in the formulae (1) to (3), Ar¹ is a 1,4-phenylene group, a 2,6-naphthalenediyl group or a 4,4'-biphenylylene group, Ar² is a 1,4-phenylene group, a 1,3-phenylene group or a 2,6-naphthalenediyl group, Ar³ is a 1,4-phenylene group or a 1,3-phenylene group, X is -NH-, Y is -O- or -NH-, and hydrogen atoms in Ar¹,Ar² or Ar³ may be each independently substituted with a halogen atom, an alkyl group having a carbon number of 1 to 10 or an aryl group having a carbon number of 6 to 20.

7. A method for producing a liquid crystal polyester solution composition, the method comprising dissolving a liquid crystal polyester powder of any one of claims 1 to 6 in an aprotic solvent to obtain the liquid crystal polyester solution composition.
